# EUROPÄISCHE PATENTSCHRIFT

(11) **EP 0 744 397 B1**
(45) Veröffentlichungstag und Bekanntmachung des Hinweises auf die Patenterteilung: **03.05.2000**
(21) Anmeldenummer: 96107622.1
(22) Anmeldetag: 14.05.1996
(51) Int. Cl.: C07C 279/22, A61K 31/155, A61K 31/16

(54) **Fluorphenylsubstituierte Alkenylcarbonsäureguanidine als Natriumprotonen-Antiporter-Inhibitoren, Verfahren zu ihrer Herstellung, ihre Verwendung als Medikament oder Diagnostikum sowie sie enthaltendes Medikament**
Fluorophenyl substituted alkenyl carboxylic acid guanidines as Na+/H+ exchange inhibitors, process for their preparation, their use as medicinal or diagnostic agent as well as a medicament containing them
Guanidines d'acides alcénylcarboxyliques substituées par un groupe fluorophényle comme inhibiteurs de l'échange Na+/H+, leur procédé de préparation, leur utilisation comme médicament ou agent diagnostique ainsi que médicament les contenant

(30) Priorität: 22.05.1995 DE 19518796
(43) Veröffentlichungstag der Anmeldung: 27.11.1996
(73) Patentinhaber: HOECHST AKTIENGESELLSCHAFT, 65929 Frankfurt am Main (DE)
(72) Erfinder: Schwark, Jan-Robert, Dr., 65929 Frankfurt (DE); Lang, Hans Jochen, Dr., 65719 Hofheim (DE); Kleemann, Heinz-Werner, Dr., 65474 Bischofsheim (DE); Weichert, Andreas, Dr., 63329 Egelsbach (DE); Scholz, Wolfgang, Dr., 65760 Eschborn (DE); Albus, Udo, Dr., 61197 Florstadt (DE)

(56) Entgegenhaltungen:
- EP-A- 0 612 723
- EP-A- 0 640 588
- US-A- 2 734 904

## Beschreibung

Die Erfindung betrifft Fluorphenylgruppen tragende Alkenylcarbonsäure-guanidide der Formel I 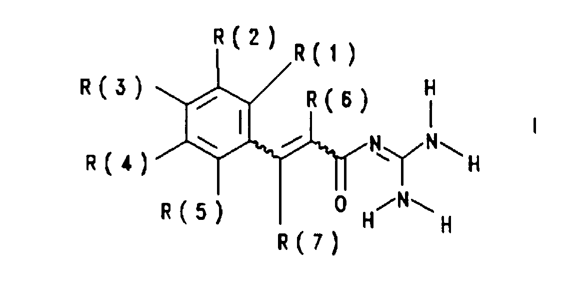 worin bedeuten:
- R(6): Wasserstoff, (C₁-C₈)-Alkyl, (C₃-C₈)-Cycloalkyl oder Phenyl, wobei die Phenylgruppe nicht substituiert oder substituiert ist mit 1 - 3 Substituenten ausgewählt aus der Gruppe bestehend aus F, Cl, CF₃, Methyl, Methoxy und NR(9)R(10);
R(9) und R(10) Wasserstoff, (C₁-C₄)-Alkyl oder (C₁-C₄)-Perfluoralkyl;
- R(7): unabhängig wie R(6) definiert;
- R(1), R(2), R(3), R(4) und R(5): unabhängig voneinander Wasserstoff oder F;
wobei jedoch mindestens einer der Reste R(1), R(2), R(3), R(4) und R(5) Fluor sein muß;
sowie deren pharmazeutisch verträgliche Salze.

Bevorzugt sind Verbindungen der Formel I, in der bedeuten:
- R(6): Wasserstoff, (C₁-C₄)-Alkyl oder (C₃-C₆)-Cycloalkyl;
- R(7): unabhängig wie R(6) definiert;
- R(1), R(2), R(3), R(4) und R(5): unabhängig voneinander Wasserstoff oder F;
wobei jedoch mindestens einer der Reste R(1), R(2), R(3), R(4) und R(5) Fluor sein muß;
sowie deren pharmazeutisch verträgliche Salze.

Besonders bevorzugt sind Verbindungen der Formel I, in der bedeuten:
- R(6): Wasserstoff oder CH₃;
- R(7): Wasserstoff;
- R(1), R(2), R(3), R(4) und R(5): unabhängig voneinander Wasserstoff oder F;
wobei jedoch mindestens einer der Reste R(1), R(2), R(3), R(4) und R(5) Fluor sein muß;
sowie deren pharmazeutisch verträgliche Salze.

Enthält die Verbindungen der Formel I ein oder mehrere Asymmetriezentren, so können diese sowohl S als auch R konfiguriert sein. Die Verbindungen können als optische Isomere, als Diastereomere, als Racemate oder als Gemische derselben vorliegen.

Die Doppelbindungsgeometrie der Verbindungen der Formel I kann sowohl E als auch Z sein. Die Verbindungen können als Doppelbindungsisomere im Gemisch vorliegen.

Die bezeichneten Alkylreste können sowohl geradkettig als auch verzweigt vorliegen.

Die Erfindung betrifft weiterhin ein Verfahren zur Herstellung der Verbindung I, dadurch gekennzeichnet, daß man
eine Verbindung der Formel II 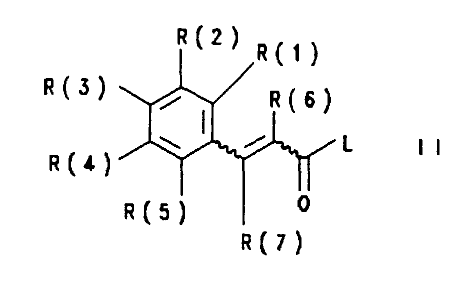 mit Guanidin umsetzt, worin R(1) bis R(7) die angegebene Bedeutung besitzen und L für eine leicht nucleophil substituierbare leaving group steht.

Die aktivierten Säurederivate der Formel II, worin L eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise 1-Imidazolyl, bedeutet, erhält man vorteilhaft in an sich bekannter Weise aus den zugrundeliegenden Carbonsäurechloriden (Formel II, L = Cl), die man ihrerseits wiederum in an sich bekannter Weise aus den zugrundeliegenden Carbonsäuren (Formel II, L = OH) beispielsweise mit Thionylchlorid herstellen kann.

Neben den Carbonsäurechloriden der Formel II (L = Cl) lassen sich auch weitere aktivierte Säurederivate der Formel II in an sich bekannter Weise direkt aus den zugrundeliegenden Benzoesäure-derivaten (Formel II, L = OH) herstellen, wie beispielsweise die Methylester der Formel II mit L = OCH₃ durch Behandeln mit gasförmigem HCl in Methanol, die Imidazolide der Formel II durch Behandeln mit Carbonyldiimidazol [L = 1-Imidazolyl, Staab, Angew. Chem. Int. Ed. Engl. 1, 351 -367 (1962)], die gemischten Anhydride II mit Cl-COOC₂H₅ oder Tosylchlorid in Gegenwart von Triethylamin in einem inerten Lösungsmittel, wie auch die Aktivierungen von Benzoesäuren mit Dicyclohexylcarbodiimid (DCC) oder mit O-[(Cyano(ethoxycarbonyl)methylen)amino]-1,1,3,3-tetramethyluronium-tetrafluoborat ("TOTU") [Proceedings of the 21. European Peptide Symposium, Peptides 1990, Editors E. Giralt and D. Andreu, Escom, Leiden, 1991]. Eine Reihe geeigneter Methoden zur Herstellung von aktivierten Carbonsäurederivaten der Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel II mit Guanidin erfolgt in an sich bekannter Weise in einem protischen oder aprotischen polaren aber inerten organischen Lösungsmittel. Dabei haben sich bei der Umsetzung der Benzoesäuremethylester (II, L = OMe) mit Guanidin Methanol, Isopropanol oder THF von 20°C bis zur Siedetemperatur dieser Lösungsmittel bewährt. Bei den meisten Umsetzungen von Verbindungen II mit salzfreiem Guanidin wurde vorteilhaft in aprotischen inerten Lösungsmitteln wie THF, Dimethoxyethan, Dioxan gearbeitet. Aber auch Wasser kann unter Gebrauch einer Base wie beispielsweise NaOH als Lösungsmittel bei der Umsetzung von II mit Guanidin verwendet werden.

Wenn L = Cl bedeutet, arbeitet man vorteilhaft unter Zusatz eines Säurefängers, z. B. in Form von überschüssigem Guanidin zur Abbindung der Halogenwasserstoffsäure.

Ein Teil der zugrundeliegenden Benzoesäurederivate der Formel II ist bekannt und in der Literatur beschrieben. Die unbekannten Verbindungen der Formel II können nach literaturbekannten Methoden hergestellt werden. Die erhaltenen Alkenylcarbonsäuren werden nach einer der oben beschriebenen Verfahrensvarianten zu erfindungsgemäßen Verbindungen I umgesetzt.

Die Einführung einiger Substituenten gelingt durch literaturbekannte Methoden des Palladium-vermittelten cross-couplings von Arylhalogeniden bzw. Aryltriflaten mit beispielsweise Organostannanen, Organoboronsäuren oder Organoboranen oder Organokupfer- bzw. -zink-verbindungen.

Carbonsäureguanidine I sind im allgemeinen schwache Basen und können Säure unter Bildung von Salzen binden. Als Säureadditionssalze kommen Salze aller pharmakologisch verträglichen Säuren infrage, beispielsweise Halogenide, insbesondere Hydrochloride, Lactate, Sulfate, Citrate, Tartrate, Acetate, Phosphate, Methylsulfonate, p-Toluolsulfonate.

Die Verbindungen I sind substituierte Acylguanidine. Prominentester Vertreter der Acylguanidine ist das Pyrazinderivat Amilorid, das als kaliumsparendes Diuretikum in der Therapie Verwendung findet. Zahlreiche weitere Verbindungen vom Amilorid-Typ werden in der Literatur beschrieben, wie beispielsweise Dimethylamilorid oder Ethylisopropylamilorid. 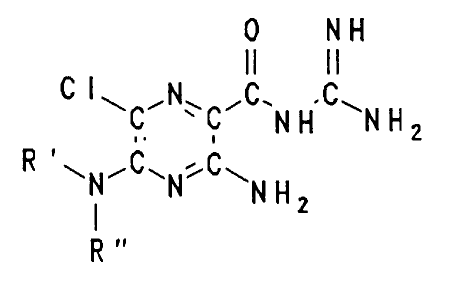
Amilorid: R',R'' = H
Dimethylamilorid: R',R" = CH₃
Ethylisopropylamilorid: R'= C₂H₅, R'' = CH(CH₃)₂

Darüberhinaus sind Untersuchungen bekannt geworden, die auf antiarrhythmische Eigenschaften von Amilorid hinweisen (Circulation 79, 1257 - 63 (1989). Einer breiten Anwendung als Antiarrhythmikum steht jedoch entgegen, daß dieser Effekt nur schwach ausgeprägt ist und von einer blutdrucksenkenden und saluretischen Wirkung begleitet auftritt und diese Nebenwirkungen bei der Behandlung von Herz-Rhythmusstörungen unerwünscht sind.

Hinweise auf antiarrhythmische Eigenschaften des Amilorids wurden auch bei Experimenten an isolierten Tierherzen erhalten (Eur. Heart J. 9 (suppl.1): 167 (1988) (book of abstracts). So wurde beispielsweise an Rattenherzen gefunden, daß ein künstlich ausgelöstes Kammerflimmern durch Amilorid völlig unterdrückt werden konnte. Noch potenter als Amilorid war in diesem Modell das oben erwähnte Amiloridderivat Ethylisopropylamilorid.

Aus WO 84/ 00875 sind Zimtsäureguanidide bekannt, (Rₐ und R_{c}, bzw. R_{b} und R_{d} = Doppelbindung; R(1) = substituiertes Phenyl); jedoch sind diese in allen Fällen am Guanidin zusätzlich mit Alkylgruppen substituiert, weshalb sie keine NHE-Inhibition zeigen sollten. Außerdem ist als Substituent am Phenylkern nur allgemein Halogen erwähnt, das zwar als "alle vier Halogene" definiert ist, jedoch findet sich kein Individuum mit Fluor-Substitution.

Aus US 2,734,904 (erteilt 1956) sind Zimtsäureguanidide bekannt (R = substituiertes Phenyl, Alkyl = Alkenylen), jedoch sind als Halogen-Substituenten am Phenylkern nur Chlor, Brom, Jod und nicht Fluor beschrieben; im Anspruch wird Fluor ausgeschlossen (Halogene mit einer Ordnungszahl > 9 und < 53).

Aus der EP 640 588 sind Benzoylguanidine bekannt, bei welchen die Acylguanidingruppe direkt am Phenylkern sitzt; Verbindungen mit einer ungesättigten Gruppe zwischen der Acyl- und der Phenylgruppe sind dort nicht beschrieben.

In der deutschen Offenlegungsschrift 44 21 536.3 werden Zimtsäureguanidide vorgeschlagen (x = 0, y = 0), jedoch muß einer der Substituenten R(1), R(2), R(4), R(5), R(C) oder R(D) eine Perfluoralkylgruppe sein.

Es war daher überraschend, daß die erfindungsgemäßen Verbindungen keine unerwünschten und nachteiligen salidiuretischen, jedoch sehr gute antiarrhythmische Eigenschaften aufweisen. Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge Inhibition des zellulären Na⁺/H⁺-Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüberhinaus eignen sich die erfindungsgemäßen Verbindungen der Formel I ebenfalls zur Behandlungen von Formen des Schocks, wie beispielweise des allergischen, cardiogenen, hypovolämischen und des bakteriellen Schocks.

Darüberhinaus zeichnen sich die erfindungsgemäßen Verbindungen der Formel I durch starke inhibierende Wirkung auf die Proliferationen von Zellen, beispielsweise der Fibroblasten- Zellproliferation und der Proliferation der glatten Gefäßmuskelzellen, aus. Deshalb kommen die Verbindungen der Formel I als wertvolle Therapeutika für Krankheiten infrage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters (Na⁺/H⁺-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindungen der Formel I für die präventive Therapie zur Verhinderung der Genese des Bluthochdrucks, beispielweise der essentiellen Hypertonie, geeignet.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers. Im Durchschnitt beträgt die tägliche Dosis einer Verbindung der Formel I bei einem etwa 75 kg schweren Patienten mindestens 0,001 mg/kg, vorzugsweise 0,01 mg/kg, bis höchstens 10 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z. B. bis zu 4 Einzeldosen pro Tag. Insbesondere bei i.v. Anwendung, etwa bei einem Infarktpatienten auf der Intensivstation können bis zu 200 mg pro Tag notwendig werden.

### Liste der Abkürzungen:

- MeOH: Methanol
- DMF: N,N-Dimethylformamid
- El: electron impact
- DCI: Desorption-Chemical Ionisation
- RT: Raumtemperatur
- EE: Ethylacetat (EtOAc)
- mp: Schmelzpunkt
- HEP: n-Heptan
- DME: Dimethoxyethan
- ES: Elektronenspray
- FAB: Fast Atom Bombardment
- CH₂Cl₂: Dichlormethan
- THF: Tetrahydrofuran
- eq.: Äquivalent

### Experimenteller Teil

### Allgemeine Vorschriften zur Herstellung von Alkenylcarbonsäure-guanididen (I)

### Variante 1 A: aus Alkenylcarbonsäuren (II, L = OH)

1.0 eq. des Carbonsäurederivates der Formel II löst bzw. suspendiert man in wasserfreiem THF ( 5 ml/mmol ) und versetzt sodann mit 1.1 eq. Carbonyldiimidazol. Nach dem Rühren über 2 Stunden bei RT werden 5.0 eq. Guanidin in die Reaktionslösung eingetragen. Nach dem Rühren über Nacht destilliert man das THF unter vermindertem Druck (im Rotationsverdampfer) ab, versetzt mit Wasser, stellt mit 2 N HCl auf pH 6 bis 7 und filtriert das entsprechende Guanidid (Formel I) ab. Die so erhaltenen Carbonsäureguanidine können durch Behandeln mit wäßriger, methanolischer oder etherischer Salzsäure oder anderen pharmakologisch verträglichen Säuren in die entsprechenden Salze übergeführt werden.

### Variante 1 B: aus Alkenylcarbonsäure-alkylestern (II, L = O-Alkyl)

1.0 eq. des Carbonsäure-alkylesters der Formel II sowie 5.0 eq. Guanidin (freie Base) werden in Isopropanol gelöst oder in THF suspendiert und bis zum vollständigen Umsatz (Dünnschichtkontrolle) unter Rückfluß gekocht (typische Reaktionszeit 2 bis 5 h). Das Lösungsmittel wird unter vermindertem Druck (Rotavapor) abdestilliert in EE aufgenommen und 3 x mit NaHCO₃-Lösung gewaschen. Es wird über Na₂SO₄ getrocknet, das Lösungsmittel im Vakuum abdestilliert und an Kieselgel mit einem geeigneten Laufmittel, z. B. EE/MeOH 5 : 1 chromatographiert.
(Salzbildung vergleiche Variante A)

### Beispiel 1: E-3-(3-Fluorphenyl)acrylsäureguanidid-Hydrochlorid

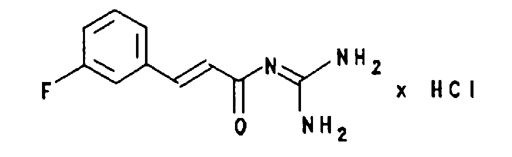

wurde gemäß Variante 1 A aus meta-Fluorzimtsäure dargestellt.
mp 148°C MS:208 (M+1)⁺

### Beispiel 2: E-3-(2,5-Difluorphenyl)acrylsäureguanidid-Hydrochlorid

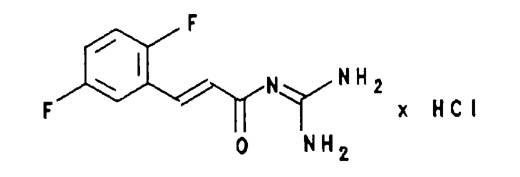

Wurde gemäß Variante 1 A aus 2,5-Difluorzimtsäure dargestellt.
mp 230°C MS:226 (M+1)⁺

### Beispiel 3: E-3-(3,5-Difluorphenyl)acrylsäureguanidid-Hydrochlorid

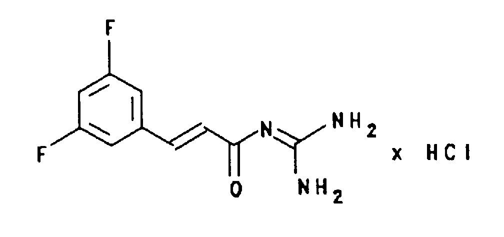

wurde gemäß Variante 1 A aus 3,5-Difluorzimtsäure dargestellt.
mp 235°C MS: 226 (M+1)⁺

### Beispiel 4: E-3-(2-Fluorphenyl)acrylsäureguanidid-Hydrochlorid

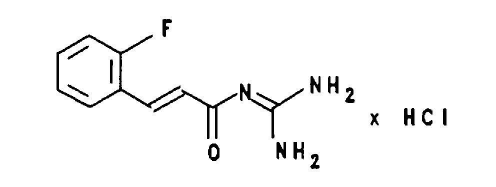

wurde gemäß Variante 1 A aus ortho-Fluorzimtsäure dargestellt.
mp 243°C MS: 208 (M + 1)⁺

### Beispiel 5: E-3-(3,5-Difluorphenyl)-2-methyl-acrylsäureguanidid-Hydrochlorid

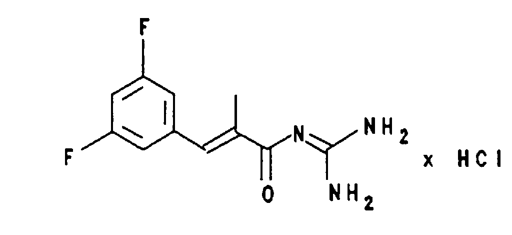

5 a) 1 eq. 2-Phosphonopropionsäuretriethylester wurde bei 0°C mit 1 eq. n-Butyllithium in Hexan deprotoniert und anschließend bei RT mit 1 eq. 3,5-Difluorbenzaldehyd versetzt. Nach vollständiger Reaktion des Aldehyds wurde mit Wasser aufgearbeitet und dreimal mit Toluol ausgeschüttelt. Nach Trocknen der vereinigten organischen Phasen über Magnesiumsulfat wurde das Lösungsmittel im Vakuum entfernt und das verbleibende Rohprodukt chromatographisch an Kieselgel mit EE/HEP-Gemischen als Eluens getrennt. Man isolierte E-3-(3,5-Difluorphenyl)-2-methyl-acrylsäureethylester.

Der Ester aus 5 a) wurde gemäß Variante 1 B zum E-3-(3,5-Difluorphenyl)-2-methyl-acrylsäureguanidid umgesetzt und ins Hydrochlorid überführt.
mp 178°C MS: 240 (M + 1)⁺

### Beispiel 6: E-3-(2-Fluorphenyl)-2-methyl-acrylsäureguanidid-Hydrochlorid

E-3-(2-Fluorphenyl)-2-methyl-acrylsäureguanidid wurde in Analogie zu Beispiel 5 ausgehend von 2-Fluorbenzaldehyd synthetisiert und als Hydrochlorid isoliert.
mp 130°C MS: 222 (M+1)⁺

### Beispiel 7: E-3-(4-Fluorphenyl)-2-methyl-acrylsäureguanidid-Hydrochlorid

E-3-(4-Fluorphenyl)-2-methyl-acrylsäureguanidid wurde in Analogie zu Beispiel 5 ausgehend von 4-Fluorbenzaldehyd synthetisiert und als Hydrochlorid isoliert.
mp 111°C MS: 222 (M+1)⁺

### Beispiel 8: E-3-(2,3,6-Trifluorphenyl)-2-methyl-acrylsäureguanidid-Hydrochlorid

E-3-(2,3,6-Trifluorphenyl)-2-methyl-acrylsäureguanidid wurde in Analogie zu Beispiel 5 ausgehend von 2,3,6-Trifluorbenzaldehyd synthetisiert und als Hydrochlorid isoliert.
mp 152°C MS: 258 (M+1)⁺

### Beispiel 9: E-3-(2,3,5,6-Tetrafluorphenyl)-2-methyl-acrylsäureguanidid-Hydrochlorid

E-3-(2,3,5,6-Tetrafluorphenyl)-2-methyl-acrylsäureguanidid wurde in Analogie zu Beispiel 5 ausgehend von 2,3,5,6-Tetrafluorbenzaldehyd synthetisiert und als Hydrochlorid isoliert.
mp 138°C MS: 276 (M+1)⁺

### Beispiel 10: E-3-(2,3,4,5,6-Pentafluorphenyl)-2-methyl-acrylsäureguanidid-Hydrochlorid

E-3-(2,3,4,5,6-Pentafluorphenyl)-2-methyl-acrylsäureguanidid wurde in Analogie zu Beispiel 5 ausgehend von 2,3,4,5,6-Pentafluorbenzaldehyd synthetisiert und als Hydrochlorid isoliert.
mp 140°C MS: 294 (M+1)⁺

### Beispiel 11: E-3-(2,4,6-Trifluorphenyl)-2-methyl-acrylsäureguanidid-Hydrochlorid

E-3-(2,4,6-Trifluorphenyl)-2-methyl-acrylsäureguanidid wurde in Analogie zu Beispiel 5 ausgehend von 2,4,6-Trifluorbenzaldehyd synthetisiert und als Hydrochlorid isoliert.
mp 155°C MS: 258 (M+1)⁺

### Beispiel 12: E-3-(2,6-Difluorphenyl)-2-methyl-acrylsäureguanidid-Hydrochlorid

E-3-(2,6-Difluorphenyl)-2-methyl-acrylsäureguanidid wurde in Analogie zu Beispiel 5 ausgehend von 2,6-Difluorbenzaldehyd synthetisiert und als Hydrochlorid isoliert.
mp 155°C MS: 240 (M+1)⁺

### Pharmakologische Daten:

### Inhibitoren des Na⁺/H⁺-Exchangers von Kaninchenerythozyten:

Weiße Neuseeland-Kaninchen (Ivanovas) erhielten eine Standard-Diät mit 2% Cholesterin für sechs Wochen, um den Na⁺/H⁺-Austausch zu aktivieren und so den Na⁺-Influx in die Erythrozyten via Na⁺/H⁺-Austausch flammenphotometrisch bestimmen zu können. Das Blut wurde den Ohrarterien entnommen und durch 25 IE/ml Kalium-Heparin ungerinnbar gemacht. Ein Teil jeder Probe wurde zur Doppelbestimmung des Hämatokrits durch Zentrifugation benutzt. Aliquots von jeweils 100 µl dienten zur Messung des Na⁺-Ausgangsgehalts der Erythrozyten.

Um den Amilorid-sensitiven Natrium-Influx zu bestimmen, wurden 100 µl jeder Blutprobe in jeweils 5 ml eines hyperosmolaren Salz-Sucrose-Mediums (mmol/l: 140 NaCl, 3 KCl, 150 Sucrose, 0,1 Ouabain, 20 Tris-hydroxymethylaminomethan) bei pH 7,4 und 37°C inkubiert. Die Erythrozyten wurden danach dreimal mit eiskalter MgCl₂-Ouabain-Lösung (mmol/l: 112 MgCl₂, 0,1 Ouabain) gewaschen und in 2,0 ml destilliertem Wasser hämolysiert. Der intrazelluläre Natriumgehalt wurde flammenphotometrisch bestimmt.

Der Na⁺-Nettoinflux wurde aus der Differenz zwischen Natrium-Ausgangswerten und dem Natriumgehalt der Erythrozyten nach Inkubation errechnet. Der Amilorid-hemmbare Natrium-Influx ergab sich aus der Differenz des Natriumgehalts der Erythrozyten nach Inkubation mit und ohne Amilorid 3 x 10⁻⁴ mol/l. Auf diese Weise wurde auch bei den erfindungsgemäßen Verbindungen verfahren.

Ergebnisse zur Inhibition des Na⁺/H⁺-Exchangers:

| Beispiel | IC₅₀ [ mol/l] |
|---|---|
| 2 | < 1 |
| 3 | < 1 |
| 4 | < 1 |
| 5 | < 1 |
| 9 | < 1 |

## Patentansprüche

1. Fluorphenylgruppen tragende Alkenylcarbonsäure-guanidide der Formel I 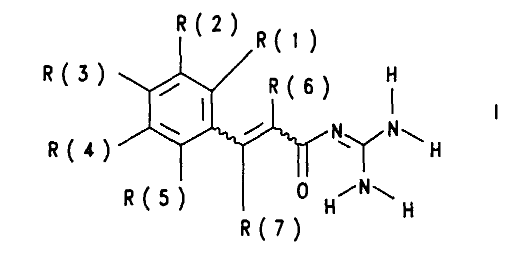 worin bedeuten:
R(6) Wasserstoff, (C₁-C₈)-Alkyl, (C₃-C₈)-Cycloalkyl oder Phenyl, wobei die Phenylgruppe nicht substituiert oder substituiert ist mit 1 - 3 Substituenten ausgewählt aus der Gruppe bestehend aus F, Cl, CF₃, Methyl, Methoxy und NR(9)R(10);
R(9) und R(10) Wasserstoff, (C₁-C₄)-Alkyl oder (C₁-C₄)-Perfluoralkyl;
R(7) unabhängig wie R(6) definiert;
R(1), R(2), R(3), R(4) und R(5) unabhängig voneinander Wasserstoff oder F;
wobei jedoch mindestens einer der Reste R(1), R(2), R(3), R(4) und R(5) Fluor sein muß;
sowie deren pharmazeutisch verträgliche Salze.

2. Verbindung der Formel I nach Anspruch 1, dadurch gekennzeichnet, daß darin bedeuten:
R(6) Wasserstoff, (C₁-C₄)-Alkyl oder (C₃-C₆)-Cycloalkyl;
R(7) unabhängig wie R(6) definiert;
R(1), R(2), R(3), R(4) und R(5) unabhängig voneinander Wasserstoff oder F;
wobei jedoch mindestens einer der Reste R(1), R(2), R(3), R(4) und R(5) Fluor sein muß.

3. Verbindung der Formel I nach Ansprüchen 1 oder 2, in der bedeuten:
R(6) Wasserstoff oder CH₃;
R(7) Wasserstoff;
R(1), R(2), R(3), R(4) und R(5) unabhängig voneinander Wasserstoff oder F;
wobei jedoch mindestens einer der Reste R(1), R(2), R(3), R(4) und R(5) Fluor sein muß.

4. Verfahren zur Herstellung einer Verbindung I nach Anspruch 1, dadurch gekennzeichnet, daß man
ein Verbindung der Formel II 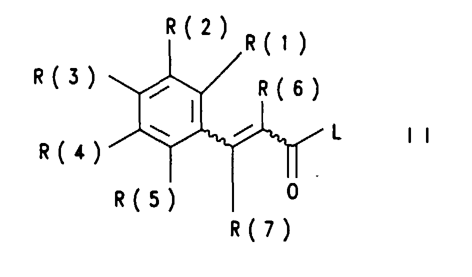 mit Guanidin umsetzt, worin R(1) bis R(7) die angegebene Bedeutung besitzen und L für Methoxy, Phenoxy, Phenylthio, Methylthio, 2-Pyridylthio, Imdiazolyl, -OCOOEthyl, -O-SO₂-Tolyl, -O-C(=N-Cyclohexyl)-(NH-Cyclohexyl) oder -O-C⁺[N(CH₃)₂][N(CH₃)₂] steht.

5. Verwendung einer Verbindung 1 nach Anspruch 1 zur Herstellung eines Medikaments zu Behandlung von Arrhythmien.

6. Verwendung einer Verbindung I nach Anspruch 1 zur Herstellung eines Medikaments zur Behandlung oder Prophylaxe des Herzinfarkts.

7. Verwendung einer Verbindung I nach Anspruch 1 zur Herstellung eines Medikaments zur Behandlung oder Prophylaxe der Angina Pectoris.

8. Verwendung einer Verbindung I nach Anspruch 1 zur Herstellung eines Medikaments zur Behandlung oder Prophylaxe von ischämischen Zuständen des Herzens.

9. Verwendung einer Verbindung I nach Anspruch 1 zur Herstellung eines Medikaments zur Behandlung oder Prophylaxe von ischämischen Zuständen des peripheren und zentralen Nervensystems und des Schlaganfalls.

10. Verwendung einer Verbindung I nach Anspruch 1 zur Herstellung eines Medikaments zur Behandlung oder Prophylaxe von ischämischen Zuständen peripherer Organe und Gliedmaßen.

11. Verwendung einer Verbindung I nach Anspruch 1 zur Herstellung eines Medikaments zur Behandlung von Schockzuständen.

12. Verwendung einer Verbindung I nach Anspruch 1 zur Herstellung eines Medikaments zum Einsatz bei chirurgischen Operationen und Organtransplantationen.

13. Verwendung einer Verbindung I nach Anspruch 1 zur Herstellung eines Medikaments zur Konservierung und Lagerung von Transplantaten für chirurgische Maßnahmen.

14. Verwendung einer Verbindung I nach Anspruch I zur Herstellung eines Medikaments zur Behandlung von Krankheiten, bei denen die Zellproliferation eine primäre oder sekundäre Ursache darstellt.

15. Arzneimittel, enthaltend eine wirksame Menge einer Verbindung I nach einem oder mehreren der Ansprüche 1 bis 3.

## Claims

1. An alkenylcarboxylic acid guanidide carrying fluorophenyl groups, of the formula I 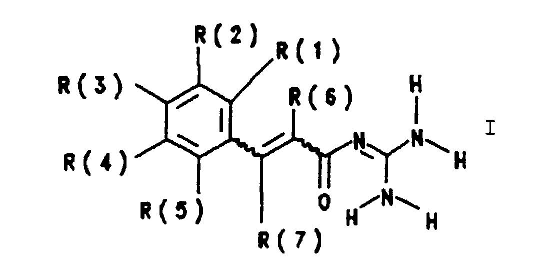 in which:
R(6) is hydrogen, (C₁-C₈)-alkyl, (C₃-C₈)-cycloalkyl or phenyl,
the phenyl group being unsubstituted or substituted by 1 - 3 substituents selected from the group comprising F, Cl, CF₃, methyl, methoxy and NR(9)R(10);
R(9) and R(10) are hydrogen, (C₁-C₄)-alkyl or (C₁-C₄)-perfluoroalkyls
R(7) is independently defined in the same way as R(6); and
R(1), R(2), R(3), R(4) and R(5) independently of one another are hydrogen or F;
it being necessary, however, for at least one of the radicals R(1), R(2), R(3), R(4) and R(5) to be fluorine;
and their pharmaceutically tolerated salts.

2. A compound of the formula I as claimed in claim 1 wherein:
R(6) is hydrogen, (C₁-C₄)-alkyl or (C₃-C₆)-cycloalkyl;
R(7) is independently defined in the same way as R(6); and
R(1), R(2), R(3), R(4) and R(5) independently of one another are hydrogen or F;
it being necessary, however, for at least one of the radicals R(1), R(2), R(3), R(4) and R(5) to be fluorine.

3. A compound of the formula I as claimed in claim 1 or 2 wherein:
R(6) is hydrogen or CH₃;
R(7) is hydrogen; and
R(1), R(2), R(3), R(4) and R(5) independently of one another are hydrogen or F;
it being necessary, however, for at least one of the radicals R(1), R(2), R(3), R(4) and R(5) to be fluorine.

4. A process for the preparation of a compound I as claimed in claim 1, which comprises reacting a compound of the formula II 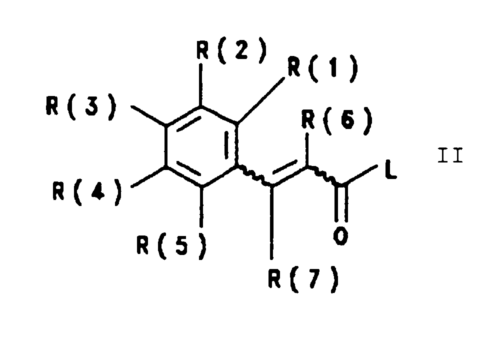 with guanidine, R(1) to R(7) being defined as indicated and L being methoxy, phenoxy, phenylthio, methylthio, 2-pyridylthio, imidazolyl, -OCOOethyl, -O-SO₂-tolyl, -O-C(=N-cyclohexyl)-(NH-cyclohexyl) or -O-C⁺[N(CH₃)₂N(CH₃)₂].

5. Use of a compound I as claimed in claim 1 for the preparation of a medicament for the treatment of - arrhythmia.

6. Use of a compound I as claimed in claim 1 for the preparation of a medicament for the treatment or prophylaxis of cardiac infarction.

7. Use of a compound I as claimed in claim 1 for the preparation of a medicament for the treatment or prophylaxis of angina pectoris.

8. Use of a compound I as claimed in claim 1 for the preparation of a medicament for the treatment or prophylaxis of ischemic heart conditions.

9. Use of a compound I as claimed in claim 1 for the preparation of a medicament for the treatment or prophylaxis of ischemic conditions of the peripheral and central nervous system and stroke.

10. Use of a compound I as claimed in claim 1 for the preparation of a medicament for the treatment or prophylaxis of ischemic conditions of peripheral organs and limbs.

11. Use of a compound I as claimed in claim 1 for the preparation of a medicament for the treatment of states of shock.

12. Use of a compound I as claimed in claim 1 for the preparation of a medicament for use in surgical operations and organ transplants.

13. Use of a compound I as claimed in claim 1 for the preparation of a medicament for the preservation and storage of transplants for surgical procedures.

14. Use of a compound I as claimed in claim 1 for the preparation of a medicament for the treatment of diseases where cell proliferation is a primary or secondary cause.

15. A drug containing an effective amount of a compound I as claimed in one or more of claims 1 to 3.

## Revendications

1. Guanidides d'acides alcénylcarboxyliques portant des groupes fluorophényle, de formule I 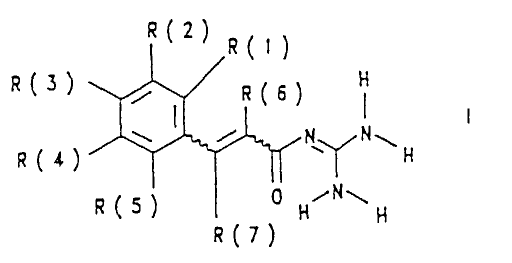 dans laquelle
R(6) représente un atome d'hydrogène ou un groupe alkyle en C₁-C₈, cycloalkyle en C₃-C₈ ou phényle,
le groupe phényle étant non substitué ou portant de 1 à 3 substituants choisis dans l'ensemble constitué par F, Cl, CF₃, le groupe méthyle, le groupe méthoxy et NR(9)R(10),
R(9) et R(10) représentant un atome d'hydrogène ou un groupe alkyle en C₁-C₄ ou perfluoroalkyle en C₁-C₄,
R(7) est, indépendamment, défini comme R(6),
R(1), R(2), R(3), R(4) et R(5) représentent, indépendamment les uns des autres, un atome d'hydrogène ou de F,
au moins l'un des radicaux R(1), R(2), R(3), R(4) et R(5) devant toutefois être un atome de fluor,
ainsi que leurs sels pharmaceutiquement acceptables.

2. Composé de formule I selon la revendication 1, caractérisé en ce que, dans ce composé:
R(6) représente un atome d'hydrogène ou un groupe alkyle en C₁-C₄ ou cycloalkyle en C₃-C₆,
R(7) est, indépendamment, défini comme R(6),
R(1), R(2), R(3), R(4) et R(5) représentent, indépendamment les uns des autres, un atome d'hydrogène ou de F,
au moins l'un des radicaux R(1), R(2), R(3), R(4) et R(5) devant toutefois être un atome de fluor.

3. Composé de formule I selon la revendication 1 ou 2, dans lequel:
R(6) représente un atome d'hydrogène ou CH₃,
R(7) représente un atome d'hydrogène,
R(1), R(2), R(3), R(4) et R(5) représentent, indépendamment les uns des autres, un atome d'hydrogène ou de F,
au moins l'un des radicaux R(1), R(2), R(3), R(4) et R(5) devant toutefois être un atome de fluor.

4. Procédé pour la préparation d'un composé I selon la revendication 1, caractérisé en ce que l'on fait réagir avec de la guanidine un composé de formule II 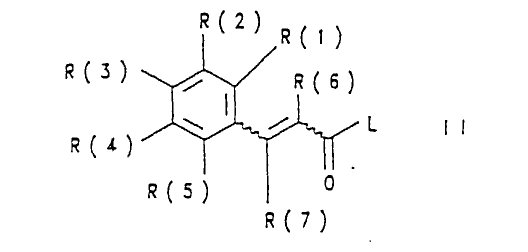 dans laquelle R(1) à R(7) ont les significations indiquées et L représente le groupe méthoxy, phénoxy, phénylthio, méthylthio, 2-pyridylthio, imidazolyle, -OCOO-éthyle, -O-SO₂-tolyle, -O-C(=N-cyclohexyl)-(NH-cyclohexyle) ou -O-C⁺[N(CH₃)₂]₂.

5. Utilisation d'un composé I selon la revendication 1, pour la fabrication d'un médicament destiné au traitement d'arythmies.

6. Utilisation d'un composé I selon la revendication 1, pour la fabrication d'un médicament destiné au traitement ou à la prophylaxie de l'infarctus du myocarde.

7. Utilisation d'un composé I selon la revendication 1, pour la fabrication d'un médicament destiné au traitement ou à la prophylaxie de l'angine de poitrine.

8. Utilisation d'un composé I selon la revendication 1, pour la fabrication d'un médicament destiné au traitement ou à la prophylaxie d'états ischémiques du coeur.

9. Utilisation d'un composé I selon la revendication 1, pour la fabrication d'un médicament destiné au traitement ou à la prophylaxie d'états ischémiques du système nerveux périphérique et central et de l'apoplexie cérébrale.

10. Utilisation d'un composé I selon la revendication 1, pour la fabrication d'un médicament destiné au traitement ou à la prophylaxie d'états ischémiques d'organes périphériques et des membres.

11. Utilisation d'un composé I selon la revendication 1, pour la fabrication d'un médicament destiné au traitement d'états de choc.

12. Utilisation d'un composé I selon la revendication 1, pour la fabrication d'un médicament destiné à l'utilisation dans des opérations chirurgicales et des greffes d'organes.

13. Utilisation d'un composé I selon la revendication 1, pour la fabrication d'un médicament destiné à la conservation et au stockage de transplants pour des opérations chirurgicales.

14. Utilisation d'un composé I selon la revendication 1, pour la fabrication d'un médicament destiné au traitement de maladies dans lesquelles la prolifération cellulaire représente une cause primaire ou secondaire.

15. Médicament, contenant une quantité efficace d'un composé I selon une ou plusieurs des revendications 1 à 3.
